(19) Europäisches Patentamt
European Patent Office
Office européen des brevets

(11) Veröffentlichungsnummer: **0 124 012**
**A2**

(12) **EUROPÄISCHE PATENTANMELDUNG**

(21) Anmeldenummer: **84104331.8**

(22) Anmeldetag: **17.04.84**

(51) Int. Cl.³: **C 07 C 49/08**, A 01 N 43/64

(30) Priorität: **30.04.83 DE 3315806**

(43) Veröffentlichungstag der Anmeldung: **07.11.84**
**Patentblatt 84/45**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Kraatz, Udo, Dr., Andreasstrasse 22a, D-5090 Leverkusen 1 (DE)**
Erfinder: **Büchel, Karl Heinz, Dr., Dabringhausener Strasse 42, D-5093 Burscheid (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen 1 (DE)**
Erfinder: **Hänssler, Gerd, Dr., Heymannstrasse 40, D-5090 Leverkusen 1 (DE)**

(54) **Phenoxytriazolyl-ketone und -carbinole, Verfahren zu ihrer Herstellung und ihre Verwendung als Fungizide.**

(57) Die Anmeldung betrifft neue Phenoxytriazolyl-ketone und -carbinole, ein Verfahren zu ihrer Herstellung und ihre Verwendung als Fungizide.

Die neuen Verbindungen der Formel

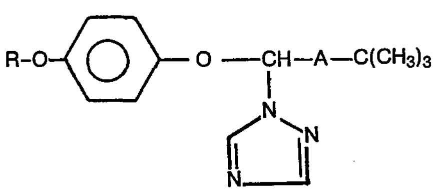

(I)

in welcher
A und R die in der Beschreibung angegebene Bedeutung besitzen,
werden erhalten, wenn man Halogen-triazolyl-pinakolin mit Phenolen zur Reaktion bringt. Die erhaltenen Wirkstoffe sind für den Gebrauch als Pflanzenschutzmittel geeignet und können mit besonders gutem Erfolg zur Bekämpfung von Getreidekrankheiten, von Venturia-Arten und von Reiskrankheiten eingesetzt werden.

EP 0 124 012 A2

BAYER AKTIENGESELLSCHAFT
Konzernverwaltung RP
Patentabteilung

5090 Leverkusen, Bayerwerk
Slr/Ke
Ia

# Phenoxytriazolyl-ketone und -carbinole, Verfahren zu ihrer Herstellung und ihre Verwendung als Fungizide

Die vorliegende Anmeldung betrifft neue Phenoxytriazolyl-ketone und -carbinole, ein Verfahren zu ihrer Herstellung und ihre Verwendung als Fungizide.

Es ist bereits bekannt geworden, daß bestimmte Phenoxytriazolyl-ketone und -carbinole, wie beispielsweise 1-(4-Chlorphenoxy)-3,3-dimethyl-1-(1,2,4-triazol-1-yl)-2-butanon, 1-(4-Bromphenoxy)-3,3-dimethyl-1-(1,2,4-triazol-1-yl)-2-butanol oder 1-(2,6-Dichlorphenoxy)-2-phenyl-1-(1,2,4-triazol-1-yl)-2-ethanon, allgemein gute fungizide Eigenschaften aufweisen (vergleiche DE-PS 22 01 063 [LeA 14 118] und DE-PS 23 24 010 [LeA 14 971]). Ebenfalls ist bereits bekannt, daß Zink-ethylen-1,2-bisdithio-carbamidat ein gutes Mittel zur Bekämpfung von pilzlichen Pflanzenkrankheiten ist (vergleiche Phytopathology 33, 1113 (1963)). Die Wirkung dieser Verbindungen ist jedoch in bestimmten Indikationsbereichen, insbesondere bei niedrigen Aufwandmengen und -konzentrationen, nicht immer voll befriedigend.

Es wurden neue Phenoxytriazolyl-ketone und -carbinole der allgemeinen Formel

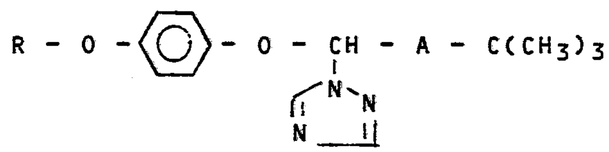

(I)

in welcher

A für die Ketogruppe oder die CH(OH)-Gruppierung steht und

R für Wasserstoff, Alkenyl, Alkinyl, Halogenalkyl, Halogenalkenyl, Alkylcarbonyl, gegebenenfalls substituiertes Benzyl oder gegebenenfalls substituiertes Phenoxymethyl steht,

sowie deren Säureadditions-Salze und Metallsalz-Komplexe gefunden.

Diejenigen Verbindungen der Formel (I), in welchen A für die CH(OH)-Gruppierung steht, besitzen zwei asymmetrische Kohlenstoffatome; sie können deshalb in den beiden geometrischen Isomeren (threo- und erythro-Form) vorliegen, die in unterschiedlichen Mengenverhältnissen anfallen können. In beiden Fällen liegen sie als optische Isomeren vor.

Weiterhin wurde gefunden, daß man die Phenoxytriazolylketone und -carbinole der allgemeinen Formel (I) erhält, wenn man Halogen-triazolyl-pinakolin der Formel

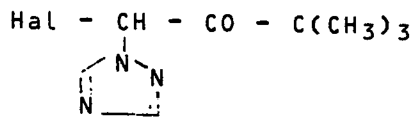 (II)

in welcher

Hal für Fluor, Chlor oder Brom steht,

mit Phenolen der Formel

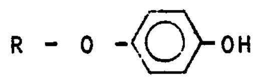 (III)

in welcher

R die oben angegebene Bedeutung hat,

in Gegenwart eines Verdünnungsmittels und in Gegenwart eines Säurebindemittels umsetzt; und gegebenenfalls noch die so erhaltenen Keto-Derivate der Formel (I) nach bekannten Methoden in üblicher Weise reduziert.

An die so erhaltenen Verbindungen der Formel (I) kann gegebenenfalls anschließend eine Säure oder ein Metallsalz addiert werden.

Die neuen Phenoxytriazolyl-ketone und -carbinole der Formel (I) weisen starke fungizide Eigenschaften auf. Dabei zeigen überraschenderweise die erfindungsgemäßen Verbindungen eine bessere fungizide Wirksamkeit als die aus dem Stand der Technik bekannten Verbindungen 1-(4-Chlorphenoxy)-3,3-dimethyl-1-(1,2,4-triazol-1-yl)-2-butanon, 1-(4-Bromphenoxy)-3,3-dimethyl-1-(1,2,4-triazol-1-yl)-2-butanol und 1-(2,6-Dichlorphenoxy)-2-phenyl-1-(1,2,4-triazol-1-yl)-2-ethanon, welches konstitutionell und wirkungsmäßig naheliegende Verbindungen sind, und als das Zinkethylen-1,2-bisdithio-carbamidat, welches ein anerkannt gutes Mittel gleicher Wirkungsrichtung ist. Die erfindunsgemäßen Stoffe stellen somit eine Bereicherung der Technik dar.

Die erfindunsggemäßen Phenoxytriazolyl-ketone und -carbinole sind durch die Formel (I) allgemein definiert. In dieser Formel steht R vorzugsweise für Wasserstoff, geradkettiges oder verzweigtes Alkenyl mit 2 bis 18 Kohlenstoffatomen, geradkettiges oder verzweigtes Alkinyl mit 2 bis 6 Kohlenstoffatomen, Halogenalkyl mit 1 oder 2 Kohlenstoffatomen und 1 bis 5 gleichen oder verschiedenen Halogenatomen, wie Fluor- und Chloratomen, Halogenalkenyl mit 2 bis 6 Kohlenstoffatomen und 1 bis 5 gleichen oder verschiedenen Halogenatomen, wie Fluor- und Chloratomen, Alkylcarbonyl mit 1 bis 4 Kohlenstoffatomen im Alkylteil, sowie für jeweils gegebenenfalls im Phenylteil einfach bis dreifach, gleich oder verschieden substituiertes Benzyl oder Phenoxymethyl, wobei als Substituenten genannt seien: Halogen, Alkyl mit 1 bis 2 Kohlenstoffatomen, Nitro sowie Halogenalkyl mit 1 bis 2 Kohlenstoffatomen und 1 bis 5 gleichen oder verschiedenen Halogenatomen, wie Fluor- und Chloratomen.

Besonders bevorzugt sind diejenigen Verbindungen der Formel (I), in denen R für Wasserstoff, geradkettiges oder verzweigtes Alkenyl mit 3 bis 12 Kohlenstoffatomen, geradkettiges oder verzweigtes Alkinyl mit 3 bis 4 Kohlenstoffatomen, Trifluormethyl, durch Chlor substituiertes Alkenyl mit 3 oder 4 Kohlenstoffatomen, Acetyl, Ethylcarbonyl sowie für jeweils gegebenenfalls im Phenylteil einfach oder zweifach, gleich oder verschieden substituiertes Benzyl oder Phenoxymethyl steht, wobei als Substituenten genannt seien: Fluor, Chlor und Methyl.

Verwendet man beispielsweise 1-Brom-3,3-dimethyl-1-(1,2,4-triazol-1-yl)-butan-2-on und 4-Allyloxyphenol als Ausgangsstoffe, so kann der Reaktionsablauf durch das folgende Formelschema wiedergegeben werden:

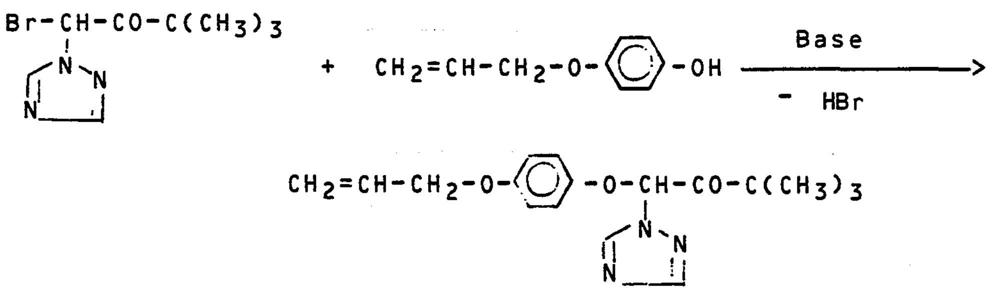


Verwendet man beispielsweise 1-(4-Allyloxyphenoxy)-3,3-dimethyl-1-(1,2,4-triazol-1-yl)-2-butanon und Natriumborhydrid als Ausgangsstoffe, so kann der Reaktionsablauf der Reduktion durch das folgende Formelschema wieder-

gegeben werden:

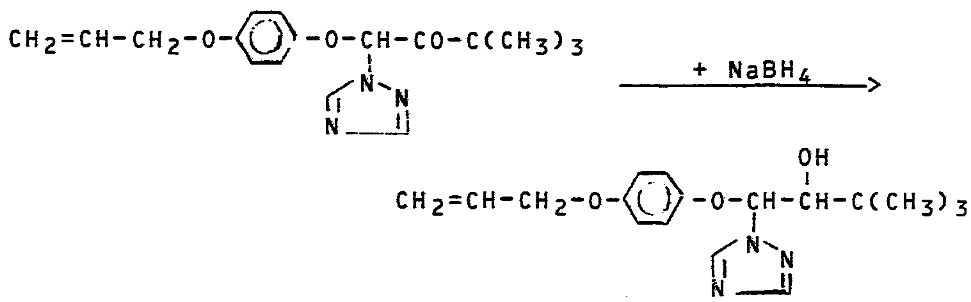


Die bei der Durchführung des erfindungsgemäßen Verfahrens als Ausgangsstoffe zu verwendenden Halogen-triazolyl-pinakoline sind durch die Formel (II) allgemein definiert. In dieser Formel steht Hal vorzugsweise für die in der Erfindungsdefinition angegebenen Bedeutungen.

Die Halogen-triazolyl-pinakoline der Formel (II) sind bekannt (vergleiche DE-OS 27 56 269 [LeA 18 565], und DE-OS 29 37 595 [LeA 19 934]). Sie können danach erhalten werden, indem man Triazolylpinakolin der Formel

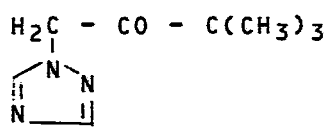

(IV)

mit Brom oder Chlor in Gegenwart eines Lösungsmittels und in Gegenwart eines Halogenwasserstoffakzeptors umsetzt und gegebenenfalls in dem erhaltenen Brom(Chlor)-triazolyl-pinakolin das Brom(Chlor) in üblicher Weise gegen Fluor austauscht.

Die außerdem für das erfindungsgemäße Verfahren als Ausgangsstoffe zu verwendenden Phenole sind durch die Formel (III) allgemein definiert. In dieser Formel steht R vorzugsweise für diejenigen Bedeutungen, die bereits im Zusammenhang mit der Beschreibung der erfindunsggemäßen Stoffe der Formel (I) vorzugsweise für diesen Rest genannt wurden.

Die Phenole der Formel (III) sind allgemein bekannte Verbindungen der organischen Chemie, bzw. können sie in allgemein üblicher Art und Weise erhalten werden.

Für die erfindungsgemäße Umsetzung kommen als Verdünnungsmittel vorzugsweise inerte organische Lösungsmittel infrage. Hierzu gehören vorzugsweise Ether, wie Diethylether; Alkohole , wie Methanol; Ketone, wie Aceton; aromatische Kohlenwasserstoffe, wie Benzol; sowie auch Dimethylsulfoxid und Dimethylformamid.
Die erfindungsgemäße Umsetzung wird in Gegenwart eines Säurebinders vorgenommen. Man kann alle üblicherweise verwendbaren anorganischen oder organischen Säurebinder zugeben, wie Alkalimetallcarbonate, beispielsweise Kaliumcarbonat oder Natriumcarbonat, Alkalimetallhydroxide, beispielsweise Natriumhydroxid, Alkalimetallalkoholate, oder wie niedere tertiäre Alkylamine, beispielsweise Triethylamin.

Die Reaktionstemperaturen können bei der Durchführung des erfindunsgemäßen Verfahrens in einem größeren Bereich variiert werden. Im allgemeinen arbeitet man zwischen 0 und 140°C, vorzugsweise zwischen 50 und 100°C.

Bei der Durchführung des erfindungsgemäßen Verfahrens setzt man auf 1 Mol der Verbindung der Formel (II) vorzugsweise 1 bis 4 Mol Phenol der Formel (III) ein. Die Isolierung der Verbindungen der Formel (I) erfolgt in üblicher Weise.

Die erfindungsgemäße Reduktion erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (I) etwa 1 Mol eines komplexen Hydrids, wie Natriumborhydrid oder Lithiumalanat ein.

Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (I) etwa 0,3 bis 2 Mol Aluminiumisopropylat ein. Zur Isolierung der reduzierten Verbindungen der Formel (I) wird das überschüssige Lösungsmittel im Vakuum entfernt und die entstandenen Aluminium-Verbindungen mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.
Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen

der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisation reinigen.

Die Verbindungen der Formel (I) können auch erhalten werden, indem man 3,3-Dimethyl-1-fluor-1-phenoxy-2-butanone(ole) der Formel

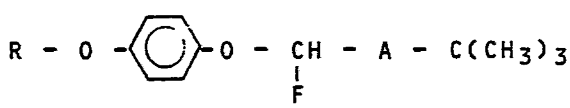

(V)

in welcher

A und R die oben angegebene Bedeutung haben,

in der Schmelze bei 100 bis 200°C mit 1,2,4-Triazol umsetzt, oder indem man die Verbindungen der Formel (V) mit Alkalimetallsalzen des 1,2,4-Triazols in einem organischen Lösungsmittel, wie beispielsweise Acetonitril, bei Temperaturen zwischen 20 und 100 °C umsetzt. Verbindungen der Formel (I), in denen A für die CH(OH)-Gruppierung steht, können auch erhalten werden, indem man zunächst die Keto-Derivate der Formel (V) in oben angegebener Art und Weise zu den entsprechenden Keto-Derivaten der Formel (I) umsetzt und anschließend die Ketogruppe wie oben beschrieben reduziert (vergleiche zu dieser Verfahrensvariante die Angaben gemäß DE-OS 30 31 846 [LeA 20 503]).

Die 3,3-Dimethyl-1-fluor-1-phenoxy-2-butanone(ole) der Formel (V) werden erhalten, indem man Halogen-ketone der Formel

$$Hal' - \underset{\underset{F}{|}}{CH} - CO - C(CH_3)_3 \qquad (VI)$$

in welcher

Hal' für Chlor oder Brom steht,

mit Phenolen der Formel (III) in Gegenwart eines inerten organischen Lösungsmittels, wie beispielsweise Aceton, und in Gegenwart eines Säurebindemittels, wie beispielsweise Kaliumcarbonat, bei Temperaturen zwischen 20 und 100°C umsetzt; und gegebenenfalls anschließend die Ketogruppe wie oben beschrieben reduziert (vergleiche hierzu die Angaben gemäß DE-OS 30 31 846 [LeA 20 503]).

Die Halogen-Ketone der Formel (VI) werden erhalten, indem man im 1-Brom(Chlor)-3,3-dimethyl-2-butanon der Formel

$$(Cl)Br-CH_2-CO-C(CH_3)_3 \qquad (VII)$$

das Brom(Chlor) in üblicher Weise gegen Fluor austauscht und im entstehenden 3,3-Dimethyl-1-fluor-2-butanon der Formel

$$F-CH_2-CO-C(CH_3)_3 \qquad (VIII)$$

eines der beiden aktiven Wasserstoffatome in üblicher Weise gegen Brom(Chlor) austauscht.

In manchen Fällen kann es sich als vorteilhaft erweisen, bestimmte Verbindungen der Formel (I) dadurch herzustellen, daß man erfindungsgemäße Verbindungen der Formel

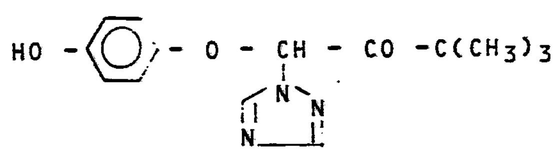
(Ia)

in bekannter Art und Weise mit einem entsprechenden Halogenid, wie beispielsweise einem Alkenylhalogenid, Alkinylhalogenid, Benzylhalogenid oder Phenoxymethylhalogenid, in Gegenwart eines inerten organischen Lösungsmittels, wie beispielsweise Aceton, und in Gegenwart eines Säurebindemittels, wie beispielsweise Kaliumcarbonat, bei Temperaturen zwischen 20 und 100°C umsetzt.

Die Verbindung der Formel (Ia) kann auch durch übliche Verseifung des entsprechenden 4-Acetoxy-Derivats mit Natronlauge erhalten werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiphoromycetes, Oomycetes, Chytridiomycetes, Zygomycetes, Ascomycetes, Basidiomycetes, Deuteromycetes.
Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.
Als Pflanzenschutzmittel können die erfindungsgemäßen Wirkstoffe mit besonders gutem Erfolg zur Bekämpfung von Getreidekrankheiten, wie Erysiphe graminis, Cochliobolus sativus, Rost, Pyrenophora teres und Schneeschimmel an Getreide, ferner von Venturia-Arten, wie Venturia inaequalis, sowie von Reiskrankheiten, wie Pellicularia sasakii und Pyricularia oryzae, eingesetzt werden.
Hervorzuheben ist, daß die erfindungsgemäßen Stoffe nicht nur eine protektive Wirkung entfalten, sondern teilweise auch systemisch wirksam sind. So gelingt es, Pflanzen gegen Pilzbefall zu schützen, wenn man die Wirkstoffe über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo -Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.
Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele:

Beispiel 1

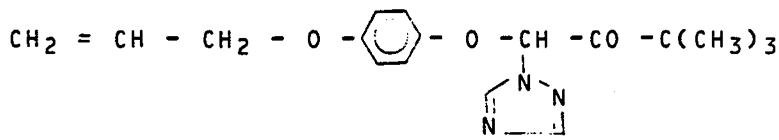

(mit Vorprodukt)

16,7g (0,1 Mol) Triazolylpinakolin und 8,2g (0,1 Mol) Natriumacetat werden in 100 ml Eisessig gelöst und bei 45°C tropfenweise mit 16,01g (0,1 Mol) Brom versetzt. Man läßt 20 Minuten bis zur vollständigen $Br_2$-Entfärbung nachrühren, gießt danach in Eiswasser und extrahiert mit Methylenchlorid. Die organische Phase wird über Natriumhydrogencarbonatlösung neutral gewaschen und bei 40°C im Vakuum eingeengt. Das zurückbleibende rohe 1-Brom-3,3-dimethyl-1-(1,2,4-triazol-1-yl)-butan-2-on wird in 40 ml Acetonitril gelöst und bei 20°C unter Rühren zu einer Lösung von 15g (0,1 Mol) 4-Allyloxyphenol sowie 10,2g (0,1 Mol) Triethylamin in 180ml Acetonitril gegeben. Dabei erfolgt eine leicht exotherme Reaktion. Man läßt das Reaktionsgemisch 30 Minuten unter Rückfluß rühren. Nach dem Abkühlen gießt man in Wasser, extrahiert mit Methylenchlorid und wäscht die organische Phase mit verdünnter Salzsäure. Nach dem Einengen im Vakuum wird der Rückstand mit Cyclohexan verrührt und abgesaugt. Man erhält 22g (70 % der Theorie) 1-(4-Allyloxyphenoxy)-3,3-dimethyl-1-(1,2,4-triazol-1-yl)-2-butanon vom Schmelzpunkt 66°C.

Beispiel 2

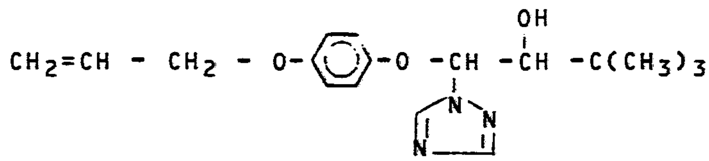

7,2g (0,023 Mol) 1-(4-Allyloxyphenoxy)-3,3-dimethyl-1-(1,2,4-triazol-1-yl)-2-butanon (Beispiel 1) werden in 80ml Methanol unter Rühren bei 20°C portionsweise mit 1g (0,027 Mol) Natriumboranat versetzt. Man läßt 1 Stunde nachrühren und engt danach im Vakuum durch Abdestillieren des Lösungsmittels ein. Der Rückstand wird zwischen Methylenchlorid/Wasser verteilt. Die organische Phase wird abgetrennt, über Natriumsulfat getrocknet und im Vakuum eingeengt. Man erhält 7g (97,2 % der Theorie) 1-(4-Allyloxyphenoxy)-3,3-dimethyl-1-(1,2,4-triazol-1-yl)-2-butanol vom Schmelzpunkt 85°C.

In analoger Weise und entsprechend den angegebenen Verfahrensbedingungen werden die in der nachfolgenden Tabelle aufgeführten Verbindungen der allgemeinen Formel

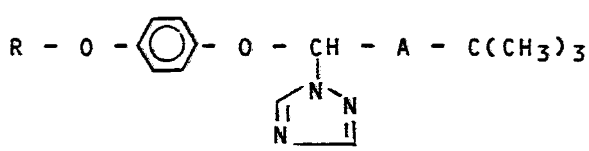 (I)

erhalten:

| Bsp.Nr. | R | A | Schmelzpunkt |
|---|---|---|---|
| 3 | $CH_3CO-$ | CO | Oel |
| 4 | H | CO | 204 |
| 5 | $CH\equiv C-CH_2-$ | CO | 97 |
| 6 | $CH\equiv C-CH_2-$ | CH(OH) | 96-100 |
| 7 | $CH_2=C(Cl)-CH_2-$ | CO | 60 |
| 8 | $CH_2=C(Cl)-CH_2-$ | CH(OH) | 68-70 |
| 9 | 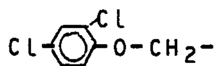 | CO | Harz |
| 10 | 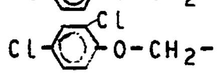 | CH(OH) | Harz |
| 11 | $(CH_3)_3C-CH_2-C(CH_3)_2-CH_2-CH=CH-CH_2-$ | CO | Oel |
| 12 | $CF_3$ | CO | 68 |
| 13 | $CF_3$ | CH(OH) | 112 |
| 14 | 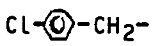 | CO | 58-60 |
| 15 | 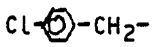 | CH(OH) | 138-40 |
| 16 | $CH_3-C(Cl)=CH-CH_2-$ | CO | Oel |
| 17 | $CH_3-C(Cl)=CH-CH_2-$ | CH(OH) | 69-72 |
| 18 | $(CH_3)_3C-CH_2-C(CH_3)_2-CH_2-CH=CH-CH_2-$ | CH(OH) | 80 |

In den nachfolgenden Beispielen werden die nachstehen angegebenen Verbindungen als Vergleichssubstanzen eingesetzt:

(A) 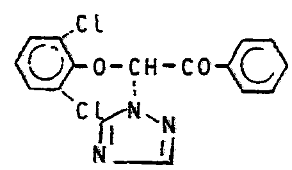

(B) 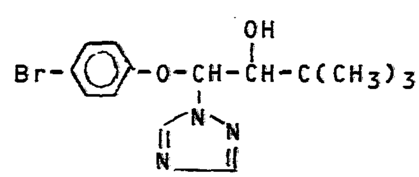

(C) 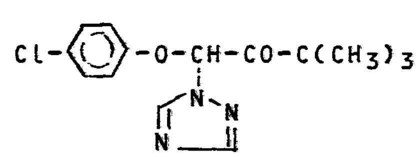

(D) 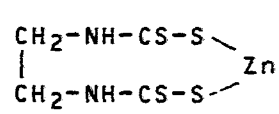

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 6, 1, 2, 7, 8, 10 und 12.

Beispiel: B

Cochliobolus sativus-Test (Gerste)/protektiv

Lösungsmittel: 100 Gewichtsteile Dimethylformamid
Emulgator : 0,25 Gewichtsteile Alkylarylpolyglkolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Ueberlegenheit in der Wirksamkeit gegenüber dem Stand der Technik zeigen bei diesem Test z.B. die Verbindungen gemäß folgender Herstellungsbeispiele: 5, 6, 1, 2, 7 und 8.

Beispiel C

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 7, 8 und 12.

Beispiel D

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäß folgender Herstellungsbeispiele: 12, 13 und 16.

Patentansprüche

1. Phenoxytriazolyl-ketone und -carbinole der allgemeinen Formel

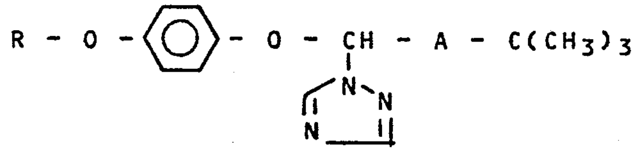

(I)

in welcher

A für die Ketogruppe oder die CH(OH)-Gruppierung steht und

R für Wasserstoff, Alkenyl, Alkinyl, Halogenalkyl, Halogenalkenyl, Alkylcarbonyl, gegebenenfalls substituiertes Benzyl oder gegebenenfalls substituiertes Phenoxymethyl steht,

sowie deren Säureadditions-Salze und Metallsalz-Komplexe

2. Verbindungen der Formel (I) in Anspruch 1, wobei

A für die Ketogruppe oder die CH(OH)-Gruppierung steht, und

R für Wasserstoff, für geradkettiges oder verzweigtes Alkenyl mit 2 bis 18 Kohlenstoffatomen, für geradkettiges oder verzweigtes Alkinyl mit 2 bis 6 Kohlenstoffatomen, für Halogenalkyl mit 1 oder 2 Kohlenstoffatomen und 1 bis 5 gleichen oder verschiedenen Halogenatomen, steht, für Halogenalkenyl mit 2 bis 6 Kohlenstoffatomen und 1 bis 5 gleichen oder verschiedenen Halogenatomen, für Alkylcarbonyl mit 1 bis 4 Kohlenstoffatomen im Alkylteil, sowie für jeweils gegebenenfalls im Phenylteil einfach bis dreifach, gleich oder verschieden durch Halogen, Alkyl mit 1 bis 2 Kohlenstoffatomen, Nitro sowie Halogenalkyl mit 1 bis 2 Kohlenstoffatomen und 1 bis 5 gleichen oder verschiedenen Halogenatomen substituiertes Benzyl oder Phenoxymethyl steht.

3. Verbindungen der Formel (I) in Anspruch 1, wobei

A für die Keto-Gruppe oder die CH(OH)-Gruppierung steht, und

R für Wasserstoff, geradkettiges oder verzweigtes Alkinyl mit 3 bis 12 Kohlenstoffatomen, geradkettiges oder verzweigtes Alkinyl mit 3 bis 4 Kohlenstoffatomen, Trifluormethyl, durch Chlor substituiertes Alkenyl mit 3 oder 4 Kohlenstoffatomen, Acetyl, Ehtylcarbonyl sowie für jeweils gegebenenfalls im Phenylteil einfach oder zweifach, gleich oder verschieden durch Fluor, Chlor und/oder Methyl substituiertes Benzyl oder Phenoxymethyl steht.

4. Verfahren zur Herstellung von Phenoxytriazolylketonen und -carbinolen der allgemeinen Formel

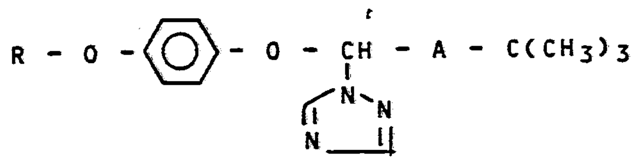

(I)

in welcher

A für die Ketogruppe oder die CH(OH)-Gruppierung steht und

R für Wasserstoff, Alkenyl, Alkinyl, Halogenalkyl, Halogenalkenyl, Alkylcarbonyl, gegebenenfalls substituiertes Benzyl oder gegebenenfalls substituiertes Phenoxymethyl steht,

dadurch gekennzeichnet, daß man Halogen-triazolyl-pinakolin der Formel

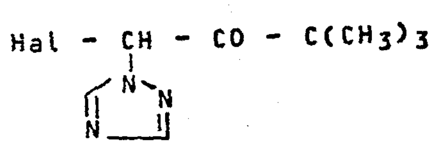 (II)

in welcher

Hal für Fluor, Chlor oder Brom steht,

mit Phenolen der Formel

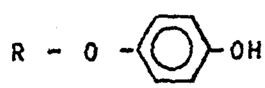 (III)

in welcher

R die oben angegebene Bedeutung hat,

in Gegenwart eines Verdünnungsmittels und in Gegenwart eines Säurebindemittels umsetzt; und gegebenenfalls noch die so erhaltenen Keto-Derivate der Formel (I) nach bekannten Methoden in üblicher Weise reduziert, und weiterhin gegebenenfalls noch an die so erhaltenen Verbindungen der Formel (I) anschließend eine Säure oder ein Metallsalz addiert.

5. Fungizide Mittel, gekennzeichnet durch einen Gehalt an mindestens einem Phenoxytriazolyl-keton oder -carbinol der Formel (I) in Ansprüchen 1 und 4.

6. Verfahren zur Bekämpfung von Pilzen, dadurch gekennzeichnet, daß man Phenoxytriazolyl-ketone oder -carbinole der Formel (I) in Ansprüchen 1 und 4, auf Pilze oder ihren Lebensraum einwirken läßt.

7. Verwendung von Phenoxytriazolyl-ketonen oder -carbinolen der Formel (I) in Ansprüchen 1 und 4, zur Bekämpfung von Pilzen.

8. Verwendung von Phenoxytriazolyl-ketonen oder -carbinolen der Formel (I) in Ansprüchen 1 und 4 als Pflanzenschutzmittel.

9. Verfahren zur Herstellung von fungiziden Mitteln, dadurch gekennzeichnet, daß man Phenoxytriazolylketone oder -carbinole der Formel (I) in Ansprüchen 1 und 4, mit Streckmitteln und/oder oberflächenaktiven Mitteln vermischt.